# EUROPEAN PATENT APPLICATION

(11) **EP 2 896 623 A1**
(43) Date of publication of application: **22.07.2015**
(21) Application number: 13834094.8
(22) Date of filing: 27.08.2013
(51) Int. Cl.: C07D 487/04, A61P 3/10, A61K 31/519

(54) **CRYSTAL OF N-[2-({2-[(2S)-2-CYANOPYRROLIDIN-1-YL]-2- OXOETHYL}AMINO)-2-METHYLPROPYL]-2-METHYLPYRAZOLO[1,5-a]PYRIMIDINE-6-CARBOXAMIDE**

(30) Priority: 28.08.2012 JP 2012187399
(71) Applicant: Sanwa Kagaku Kenkyusho Co., Ltd, Nagoya-shi Aichi 461-8631 (JP)
(72) Inventor: SAKAIRI Masao, Nagoya-shi Aichi 461-8631 (JP); INAGAKI Hideaki, Nagoya-shi Aichi 461-8631 (JP); OKA Mitsuru, Nagoya-shi Aichi 461-8631 (JP); MURASE Takayo, Nagoya-shi Aichi 461-8631 (JP)
(74) Representative: Grünecker Patent- und Rechtsanwälte PartG mbB
(86) International application number: PCT/JP2013/072793
(87) International publication number: WO 2014/034626

(57) **Abstract**

An object is to provide a crystal of anagliptin, which has a high purity and is excellent in stability, low in hygroscopicity, and has a characteristic of easily dissolving into water, and a method for producing the crystal. An anagliptin crystal showing a powder X-ray diffraction pattern substantially the same as in Fig. 1, which has peaks around at 9.8°, 17.4°, 18.6°, 25.3°, and 25.8° as diffraction angles represented by 2θ. The crystal is obtained by reacting N-(2-amino-2-methylpropyl)-2-methylpyrazolo[1,5-a]pyrimidine-6-carboxamide and (2S)-N-chloroacetyl-2-cyanopyrrolidine in an organic solvent, thereafter purifying the reaction solution by column chromatography to thus obtain an amorphia of anagliptin, and crystallizing the amorphia of anagliptin from 2-propanol.

## Description

### Technical Field

The present invention relates to a crystal of N-[2-({2-[(2S)-2-cyanopyrrolidine-1-yl]-2-oxoethyl}amino)-2-methyl propyl]-2-methylpyrazolo[1,5-a]pyrimidine-6-carboxamide (general name: anagliptin), which inhibits activity of dipeptidyl peptidase-4 (hereinafter abbreviated as DPP-4) and a method for producing the crystal.

### Background Art

Glucagon-like peptide (hereafter abbreviated as GLP-1) expresses various actions to pancreatic tissues and extrapancreatic tissues to thus allow blood sugar to decrease, and is inactivated by DPP-4 that is one kind of serine proteases. Accordingly, a pharmaceutical agent that suppresses inactivation of GLP-1 by inhibiting activity of DPP-4 was developed as a therapeutic agent for type 2 diabetes having a hypoglycemic effect. GLP-1 does not promote insulin secretion in the state of a low sugar concentration, and therefore, the present therapeutic agent is characterized as a therapeutic agent with less risk of causing low blood sugar. As this DPP-4 inhibitor, sitagliptin was first placed on the market domestically and several pharmaceutical agents are placed on the market later.

Anagliptin was also developed as a DPP-4 inhibitor, and anagliptin and its salt thereof are described in Patent Literature 1, however, its crystalline form is not described. In addition, there is a report relating to a crystal of vildagliptin having a similar structure to anagliptin (Non-Patent Literature 1), but a general method for crystallization is only described herein and the report does not describe how crystallization was actually performed; therefore, it is not clear whether crystallization with good reproducibility can be attained or not.

### Citation List

### Patent Literatures

### [Patent Literature]

[Patent Literature 1] WO No. 2004/067509

### [Non-Patent Literature]

[Non-Patent Literature 1] J. Med. Chem., 46, 2774-2789 (2003)

### Disclosure of Invention

### Problems to be Solved by the Invention

When the present inventors prepared anagliptin and hydrochloride thereof in the method described in Patent Literature 1 and analyzed the compounds in a powder X-ray diffraction method, both of the compounds were in amorphous forms. The fact that these compounds are amorphous forms means that recrystallization purification for having a high purity cannot be performed and is not favorable from the viewpoints of stability and hygroscopicity. Accordingly, an object of the present invention is to provide a crystal of anagliptin having a high purity, being excellent in stability and having a characteristic of low hygroscopicity, and a method for producing the crystal.

### Means for Solving the Problems

As a result of repeating intensive studies, the present inventors found out that a crystal of anagliptin, which has a high purity, is excellent in stability, low in hygroscopicity, and has a characteristic of easily dissolving into water and, at the same time, found out a production method suitable for crystallization of anagliptin and a method for producing a crystal of anagliptin, which is suitable for industrial manufacturing. That is, the present invention is as follows.

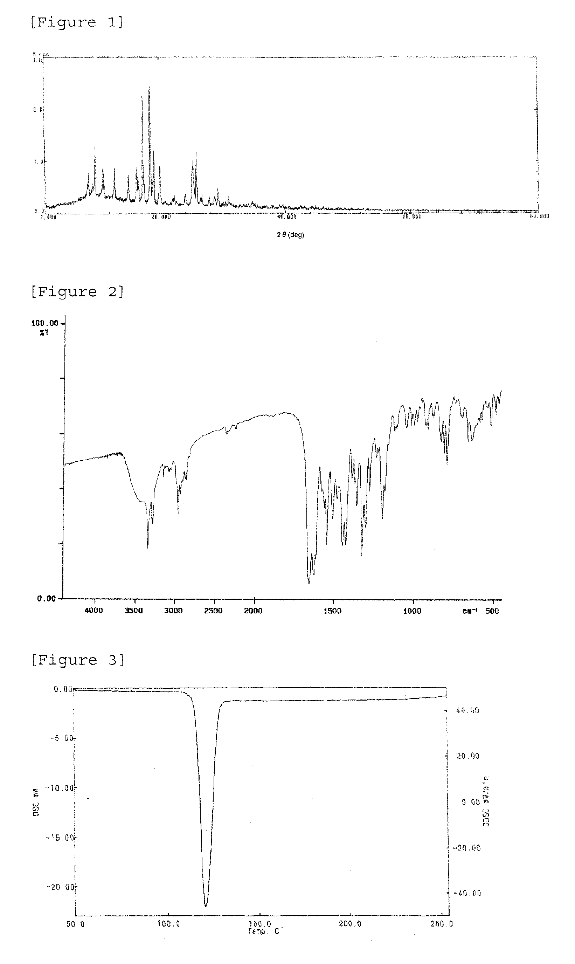

(1) A crystal of N-[2-({2-[(2S)-2-cyanopyrrolidine-1-yl]-2-oxoethyl}amino)-2-methyl propyl]-2-methylpyrazolo[1,5-a]pyrimidine-6-carboxamide (anagliptin).
(2) The crystal according to (1), which has peaks around at 9.8°, 17.4°, 18.6°, 25.3°, and 25.8° as diffraction angles represented by 2θ in a powder X-ray diffraction pattern.
(3) The crystal according to (1), wherein the diffraction angles represented by 2θ are substantially the same as the powder X-ray diffraction pattern shown in Fig. 1.

(4) The crystal according to (1), which has absorption at wavenumbers of around 3340 cm⁻¹, 1664cm⁻¹, and 1654 cm⁻¹ in an infrared absorption spectrum.
(5) The crystal according to (1), wherein the wavenumbers represented by cm⁻¹ are substantially the same as the infrared absorption spectrum shown in Fig. 2.
(6) The crystal according to (1), which has a heat absorption peak around at 120°C in differential scanning calorimetry (DSC).
(7) The crystal according to (1), wherein the heat absorption peak is substantially the same as the differential scanning calorimetry curve shown in Fig. 3.
(8) A method for producing a crystal of anagliptin, comprising reacting N-(2-amino-2-methylpropyl)-2-methylpyrazolo[1,5-a]pyrimidine-6-car boxamide and (2S)-N-chloroacetyl-2-cyanopyrrolidine in an organic solvent and thereafter crystallizing amorphia of anagliptin obtained by column chromatography purification from 2-propanol to obtain a crystal of anagliptin.
(9) A method for producing a crystal of anagliptin, comprising reacting N-(2-amino-2-methylpropyl)-2-methylpyrazolo[1,5-a]pyrimidine-6-car boxamide and (2S)-N-chloroacetyl-2-cyanopyrrolidine in an organic solvent, dissolving the obtained amorphia of anagliptin in a crystallization solvent, and thereafter crystallizing by adding a seed crystal of anagliptin to obtain a crystal of anagliptin.
(10) The method according to (8) or (9), wherein the reaction of N-(2-amino-2-methylpropyl)-2-methylpyrazolo[1,5-a]pyrimidine-6-car boxamide and (2S)-N-chloroacetyl-2-cyanopyrrolidine is carried out under the presence of alkaline carbonate and iodide.
(11) The method according to (10), wherein the alkaline carbonate is potassium carbonate and the iodide is potassium iodide.
(12) The method according to (8) or (9), wherein the organic solvent is selected from the group consisting of acetone, dichloromethane, and ethyl acetate.
(13) The method according to (8) or (9), wherein (2S)-N-chloroacetyl-2-cyanopyrrolidine is 1 to 1.2 equivalent weight with respect to N-(2-amino-2-methylpropyl)-2-methylpyrazolo[1,5-a] pyrimidine-6-carboxamide.
(14) The method according to (9), wherein the crystallization solvent is 2-propaol or ethyl acetate.
(15) The method according to (9), wherein, after the process of crystallization by adding the seed crystal of anagliptin to obtain a crystal of anagliptin, a process of crystallization by adding the seed crystal of anagliptin after concentration of the filtrate to obtain a crystal of anagliptin is further carried out plural times. Effect of Invention

The crystal of anagliptin of the present invention has a high purity, is excellent in stability, low in hygroscopicity and very easily soluble into water, and therefore extremely suitable for being used as a medical drug.

### Brief Description of Drawings

Fig. 1 shows a powder X-ray diffraction pattern of the compound 1 (crystal) (Example 1).
Fig. 2 shows an infrared absorption spectrum of the compound 1 (crystal) (Example 2).
Fig. 3 shows a differential scanning calorimetry curve of the compound 1 (crystal) (Example 2).
Fig. 4 shows a powder X-ray diffraction pattern of the compound 2 (amorphia) (Test Example 1).
Fig.5 shows a powder X-ray diffraction pattern of the compound 1 (amorphia) (Test Example 1).

### Best Mode for Carrying Out the Invention

N-[2-({2-[(2S)-2-cyanopyrrolidine-1-yl]-2-oxoethyl}amino)-2-m ethylpropyl]-2-methylpyrazolo[1,5-a]pyrimidine-6-carboxamide (anagliptin: hereinafter abbreviated as the compound 1) and its hydrochloride thereof (hereinafter abbreviated as the compound 2) are represented by the following formulas (1) and (2).

### [Compound 1]

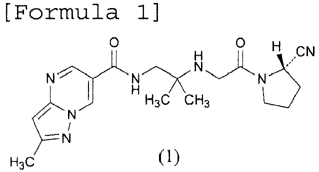

### [Compound 2]

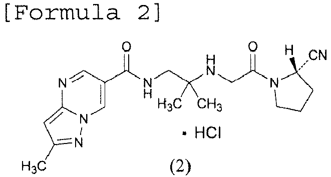

The compound 2 that is the compound of Example 2 in Patent Literature 1 (WO2004/067509) and the compound 1 that is a free base thereof can be produced by a method for producing the compound of Example 2 based on the method for producing the compound of Example 1 in Patent Literature 1. That is, a solution obtained by adding N,N'-carbonyldiimidazole (hereinafter abbreviated as CDI) to a tetrahydrofuran solution of 2-methylpyrazolo[1,5-a]pyrimidine-6-carboxylic acid to allow them to be reacted is dropped into a tetrahydrofuran solution of (S)-1-[(2-amino-1,1-dimethylethyl)aminoacetyl]pyrrolidine-2-carbon itrile-2 hydrochloride and triethylamine and the reaction solution is stirred and then the residue obtained by concentrating the reaction solution is purified by column chromatography, which thus enables the compound 1 and the compound 2 to be obtained (hereinafter referred to as the conventional method).

When the compound 1 and the compound 2 being a hydrochloride of the compound 1, which were prepared in the conventional method, were analyzed in a powder X-ray diffraction method, both of the compounds are in the amorphous form. Thus, the compound 2 in a form of a salt, which is generally considered to easily become powder, was tried to be crystallized by a cooling method using a single solvent (dichloromethane, acetonitrile, 2-propanol, ethyl acetate or acetone), but a crystal was not obtained. Next, crystallization was tried by a poor solvent method (precipitation method) using a mixed solvent (dichloromethane/ether, 2-PrOH/ether, 2-PrOH/hexane, 2-PrOH/ethyl acetate, i-BuOH/hexane, or water/acetone), but an obtained powder was not a crystal. Note that Reference Example 1 is an experiment carried out preparing the compound 2 that is a hydrochloride from a crystal of the compound 1 obtained by a modified method, which is considered to be more easily crystallized.

Furthermore, crystallization was investigated by forming the above described compound 1 into a form of a salt other than hydrochloride, that is, p-toluenesulfonate, nitrate, sulfate, 1/2 sulfate, phosphate, hydrobromate, methanesulfonate, camphorsulfonate, citrate, tartrate, malate, salicylate, phthalate, maleate, fumarate, succinate, oxalate, and malonate, in a single solvent system and a mixed solvent system, but a crystal was not able to be obtained. Note that Reference Example 2 is an experiment carried out by preparing p-toluenesulfonate from a crystal of the compound 1 obtained by a modified method described below, which is considered to be more easily crystallized.

On the other hand, the compound 1 that is a free base prepared by the conventional method was also tried to be crystallized in a cooling method using a single solvent or a mixed solvent, as shown in Reference Example 3, but all the same, a crystal was not obtained. As described above, although it was difficult that the compound 1 or salts thereof are formed into crystals, the present inventors found that when a reaction shown in the reaction procedural formula described below (formula 3) (hereinafter referred to as the modification method) was carried out, and an amorphous compound 1 obtained by purification from the reaction mixture by column chromatography was dissolved into 2-propanol and left to stand at room temperature or cooled in an ice bath, a crystal was deposited, and by filtering and drying this crystal, the inventors thus succeeded in attaining a desired crystal. Furthermore, by use of the obtained crystal as a seed crystal, a method suitable for industrial production based on the reaction procedural formula described below (Formula 3) was found. The method has advantageous effects that the crystal can be stably supplied without necessity of column chromatography and scale-up is easy. The production method will be described below.

### [Reaction Procedural Formula] Compound 3 + compound 4 → compound 1

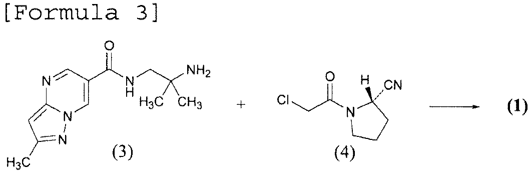

In the reaction procedural formula (Formula 3), N-(2-amino-2-methylpropyl)-2-methylpyrazolo[1,5-a]pyrimidine-6-carboxamide (hereinafter referred to as the compound 3) and (2S)-N-chloroacetyl-2-cyanopyrrolidine (hereinafter referred to as the compound 4) are allowed to be reacted in an organic solvent, thereafter purifying the reaction solution by column chromatography to obtain an amorphous compound 1, the amorphous compound 1 is crystallized by use of 2-propanol, and the crystal of the compound 1 can be thus obtained. In addition, as a suitable method for industrial production, the compound 3 and the compound 4 are allowed to be reacted in an organic solvent, the obtained amorphia of the compound 1 is dissolved into a crystallization solvent, thereafter adding the crystal of the compound 1 as a seed crystal to be crystallized and filtered, and the crystal of the compound 1 can be thus obtained. Furthermore, a filtrate after filtering the crystal is concentrated and then added with a seed crystal of the compound 1, and the crystal of the compound 1 can be thus obtained.

The compound 3 can be synthesized by reacting 2-methylpyrazolo[1,5-a]pyrimidine-6-carboxylic acid with oxalyl chloride to synthesize an acid halide and condensing the acid halide with 2-amino-2-methylpropylamine, or by condensing 2-methylpyrazolo[1,5-a]pyrimidine-6-carboxylic acid and 2-amino-2-methylpropylamine by use of a condensing agent such as CDI, as in the method described in Example 111 of an intermediate in Patent Literature 1. The above descried compound 4 can be synthesized by reacting L-proline amide and chloroacetyl chloride and then allowing the reaction product to be subjected to a dehydration reaction, similarly as in the method described in Example 1 of an intermediate in Patent Literature 1. Note that a seed crystal can be obtained by purifying from a reaction mixture obtained by reacting the above described compounds 3 and 4 to obtain an amorphous compound 1 by column chromatography and dissolving the amorphous compound 1 into 2-propanol, leaving stood at room temperature or cooling in an ice bath to be crystallized, and filtering and drying this crystal.

In the reaction of the compounds 3 and 4, the compound 4 is preferably used in an amount from 0.8 to 1.5 equivalent weight with respect to the compound 3, more preferably from 1 to 1.2 equivalent weight, and the most preferably from 1 to 1.1 equivalent weight. Furthermore, in the reaction of the compounds 3 and 4 , alkaline carbonate and iodide are preferably added. As the alkaline carbonate, examples thereof include potassium carbonate, sodium carbonate and sodium hydrogen carbonate, as the iodide, examples thereof include sodium iodide and potassium iodide, and among these examples, potassium carbonate and potassium iodide are the most preferably added. Note that alkaline carbonate is preferably used in an amount from 1 to 1.3 equivalent weight with respect to the compound 3, and iodide is preferably used in an amount from 0.5 to 1.2 equivalent weight with respect to the compound 3. The reaction temperature is preferably within the range from 5°C to 50°C, and the reaction time is preferably within the range from 1 hour to 40 hours. Examples of a reaction solvent include acetone, dichloromethane, chloroform, ethyl acetate, 2-propanol, tetrahydrofuran, and toluene, and among these solvents, acetone, dichloromethane or ethyl acetate is preferable. Examples of a crystallization solvent include 2-propnaol, ethanol, ethyl acetate, acetone, acetonitrile, tetrahydrofuran, and toluene, and among these crystallization solvents, 2-propanol or ethyl acetate is preferable. A step of obtaining a crystal of the compound 1 by crystallization by adding a seed crystal of the compound 1 after condensing the filtrate after filtering a crystal is preferably carried out plural times and generally carried out twice.

The physical properties of the crystal of the compound 1 of the present invention produced as described above are as follows, Fig. 1 shows a powder X-ray diffraction pattern, Fig. 2 shows an infrared absorption spectrum, and Fig. 3 shows a differential scanning calorimetry curve.
1. The crystal has peaks around at 9.8°, 17.4°, 18.6°, 25.3°, and 25.8° as diffraction angles represented by 2θ in the powder X-ray diffraction pattern.
2. The crystal has absorption recognized around at wave numbers of 3340 cm⁻¹, 1664 cm⁻¹, and 1654 cm⁻¹ in the infrared absorption spectrum.
3. The crystal has a heat adsorption peak around at 120°C in the differential scanning calorimetry (DSC).
4. The crystal has a degree of solubility to water of 1 g/ml or higher and the solubility is classified into "very soluble" according to the Japanese Pharmacopoeia.

The crystal of the compound 1 of the present invention has action of inhibiting an activity of DPP-4, has a high purity and is excellent in stability, and low in hygroscopicity; therefore, the crystal can be used as a drug composition. The drug composition is useful as a preventive agent or a therapeutic agent for diabetes. Regarding an administration form in the case of using the compound of the present invention as a drug, various administration forms described in the general rules for preparations in "the Japanese Pharmacopoeia" can be selected according to purposes. For example, when the present compound is prepared into a tablet form, components capable of being orally-ingested, which are generally used in this field, may be selected. For example, vehicles such as lactose, crystalline cellulose, sucrose, and potassium phosphate correspond to such components. Furthermore, various additives generally used in the field of drug formulation such as a binding agent, a disintegrating agent, a rublicant, and an agglutination inhibiting agent may be blended if desired.

The amount of the compound of the present invention contained as an active ingredient in the drug composition of the present invention is not particularly limited and is suitably selected. An amount of an active ingredient compound is suitably determined according to its usage, age, sex and other conditions of a patient and a degree of a disease, and in the case of oral administration, the amount of the compound of the present invention can be suitably administered separately once to three times within the range from 0.1 mg to 100 mg, preferably from 1 mg to 10 mg, for 1 kg of the body weight per one day. Note that, in general, an adult is orally administered in an amount of 100 mg twice per one day. However, an administration amount and an administration number are determined in consideration of related situations including a degree of a symptom to be treated and a selected administration route and, therefore, the scope of the present invention is not restricted by the ranges of the administration amount and the administration number described above.

### Examples

Hereinbelow, the present invention will be more specifically explained by way of examples, reference examples and test examples, however, the present invention is not limited thereto. Note that a powder X-ray diffraction measurement, an infrared absorption spectrum measurement, and differential scanning calorimetry were carried out in the measurement conditions described below.
[Measurement conditions of powder X-ray diffraction] Apparatus: Rigaku RAD-R, X-ray tube: Cu, tube voltage: 40 kV, tube current: 20 mL, measurement range: 2 to 80° (2θ)
[Measurement conditions of infrared absorption spectrum] Apparatus: PERKIN ELMER 1640, measurement range: 4000 to 500 cm⁻¹, resolution: 4.0 cm⁻¹, the number of scan: 16
[Measurement conditions of differential scanning calories] Apparatus: DSC220CU manufactured by Seiko Instruments Inc., measurement range: 30 to 260°C, temperature increasing rate: 10°C/min, atmosphere: N₂ 30 mL/min

### [Example 1] Crystallization of anagliptin (Compound 1)

The compound 3 (1.0 g), potassium carbonate (0.69 g) and potassium iodide (0.83 g) were suspended into acetone (25 mL), and thereto was added an acetone (17 mL) solution of the compound 4 (0.77 g, 1.1 equivalent weight with respect to the compound 3) under cooling with ice. The cooling bath was removed and the reaction solution was stirred at room temperature overnight and concentrated under reduced pressure. Chloroform (22 mL) and water (18 mL) were added to the residue to separate the solution. The aqueous layer was extracted with chloroform (18 mL), then combined with the organic layer, washed with brine (6 mL), dried over anhydrous sodium sulfate and concentrated under reduced pressure. The obtained oily substance was purified by column chromatography (elusion solvent: chloroform/methanol = 30/1 → 20/1) to obtain an amorphous compound 1 (1.2 g). When 100 mg of the amorphous compound 1 was dissolved into 2-propanol (2.4 mL) and left stood at room temperature for one day, a crystal was deposited, and this crystal was taken by filtration and dried to thus obtain a crystal of the compound 1 (36 mg) .

XRD: 9.8°, 17.4°, 18.6°, 25.3°, 25.8° (2θ).

### [Example 2] Industrial production method of crystal of anagliptin (compound 1)

The compound 3 (220 g), potassium carbonate (146 g) and potassium iodide (178 g) were suspended into acetone (2.8 L) and cooled into an ice bath. Thereto was added an acetone solution (2.8 L) containing the compound 4 (161 g, 1.04 equivalent weight with respect to the compound 3) and the reaction mixture was stirred at 5°C for 40 hours. Then, chloroform (2.8 L) and water (4.2 L) were added thereto to adjust the reaction mixture to have a pH value of 7 with 1 N hydrochloric acid. The aqueous layer was washed with chloroform and then basefied with an aqueous 8 N-sodium hydroxide solution (175 ml), and extracted with chloroform three times. The organic layer was combined and dried over anhydrous sodium sulfate and then concentrated, and the residue was added with 2-propanol (630 ml) to be dissolved. Thereto was added a seed crystal of the compound 1, the mixture was stirred at 5°C overnight, and the deposited crystal was taken by filtration. The filtrate was concentrated, and the residue was added with 2-propanol (210 ml) to be dissolved. Thereto was added a seed crystal of the compound 1, the mixture was stirred at 5°C overnight and the deposited crystal was taken by filtration. The deposited crystal was combined with the crystal obtained by filtration first and dried to thus obtain a crystal (250 g) of the compound 1. Herein, a crystal obtained in the method in Example 1 was used for the seed crystal.
mp: 117 to 119°C.
ESI-pos: m/z 384 (M+H)⁺.
ESI-neg: m/z 282 (M-H)⁻.
¹H-NMR (Methanol-d₄) δₚₚₘ: 1.17(6H,s), 2.1-2.2(2H,m), 2.2-2.3(2H,m), 2.51(3H,s), 3.41(2H,dx2), 3.52(2H,dx2), 3.6-3.7(2H,m), 4.78(1H,dd), 6.55(1H,s), 8.87(1H,d), 9.29(1H,s).
IR(KBr)cm⁻¹: 3340(N-H), 1664(C=O), 1654(C=O).
Anal. Calcd for C₁₉H₂₅N₇O₂: C,59.51; H,6.57; N,25.57; found: C,59.55; H,6.58; N,25.90.
Solubility (20°C): >lg/ml (very soluble)

### [Reference Example 1] Study of crystallization of anagliptin hydrochloride (compound 2)

The crystal (5.0 g) of the compound 1, which was obtained in Example 2, was dissolved into distilled water (15 ml) and 1 N hydrochloric acid (13 ml) was dropped thereto on an ice bath. After completion of dropping, the reaction solution was lyophilized to obtain the compound 2 (7.23 g) as an amorphia. The obtained compound 2 was dissolved by heating into a solvent A shown in Table 1 by each amount of 100 mg, and then left stood at room temperature, or added with a solvent B and left stood at room temperature. However, a crystal of the compound 2 was not able to be obtained.

**[Table 1]**

| Compound 2 | Solvent A (Amount in use) | Solvent B (Amount in use) | Result |
|---|---|---|---|
| 100 mg | Dichloromethane (1 ml) | - | No deposition |
| 100 mg | Dichloromethane (2 ml) | Ether (2 ml) | Amorphous powder |
| 100 mg | Dichloromethane (2 ml) | Hexane (2 ml) | Oil out |
| 100 mg | Acetonitrile (1 ml) | - | No deposition |
| 100 mg | 2-Propanol (2 ml) | - | Oil out |
| 100 mg | 2-Propanol (2 ml) | Ether (1 ml) | Amorphous powder |
| 100 mg | 2-Propanol (2 ml) | Hexane (1 ml) | Amorphous powder |
| 100 mg | i-Butanol (2 ml) | Hexane (2 ml) | Amorphous powder |

### [Reference Example 2] Study of crystallization of p-toluenesulfonate of anagliptin (compound 1)

The crystal (5.0g) of the compound 1, which was obtained in Example 2, was dissolved into distilled water (10 ml). Thereto was gradually added an aqueous solution (5 ml) of p-toluenesulfonic acid·H₂O (2.48 g) and the reaction mixture was stirred, then lyophilized to obtain p-toluenesulfonate (7.23 g) of the compound 1 as an amorphia. The obtained p-toluenesulfonate was dissolved by heating into a solvent A shown in Table 2 by each amount of 100 mg, and then left stood at room temperature, or added with a solvent B and left stood at room temperature. However, a crystal of p-toluenesulfonate of the compound 1 was not able to be obtained.

**[Table 2]**

| Anagliptin p-toluenesulfonate | Solvent A (Amount in use) | Solvent B (Amount in use) | Result |
|---|---|---|---|
| 100 mg | Ethanol (1 ml) | - | No deposition |
| 100 mg | 2-Propanol (1 ml) | - | No deposition |
| 100 mg | 2-Propanol (2 ml) | Ether (2 ml) | Amorphous powder |
| 100 mg | 2-Propanol (1 ml) | Hexane (2 ml) | Amorphous powder |
| 100 mg | Tetrahydrofuran (1 ml) | - | No deposition |
| 100 mg | Acetone (1 ml) | - | No deposition |
| 100 mg | Ethyl acetate (4 ml) | - | Oil out |
| 100 mg | Toluene (4 ml) | - | Oil out |
| 100 mg | Dichloromethane (1 ml) | Ether (2 ml) | Amorphous powder |
| 100 mg | Dichloromethane (1 ml) | Hexane (2 ml) | Oil out |

### [Reference Example 3] Study of crystallization of amorphous anagliptin (compound 1) obtained in conventional method

The compound 1 that is an amorphia obtained by a conventional method was dissolved by heating in a solvent A shown in Table 3 by each amount of 100 mg, thereafter forming into white turbidity in the case of adding a solvent B, and the reaction mixture was left stood at room temperature for 3 days, however, a crystal was not deposited. Thus, the reaction mixture was further left stood at 5°C for one day, however, a crystal was not deposited. Therefore, it was found that crystallization of the amorphous compound 1 obtained by a conventional method is difficult in a general method. Note that an amount in use of a solvent A* in Table 3 was set to an amount of dissolving about 40 mg of a crystal of the compound 1 from a solubility to each solvent.

**[Table 3]**

| Amorphous compound 1 | Solvent A (Amount in use) | Solvent B (Amount in use) | Result |
|---|---|---|---|
| 100 mg | Ethyl acetate (1.7 ml)* | - | No deposition |
| 100 mg | Ethanol (0.8 ml)* | - | No deposition |
| 100 mg | 2-Propanol (2.4 ml)* | | No deposition |
| 100 mg | Acetonitrile (0.2 ml)* | - | No deposition |
| 100 mg | Tetrahydrofuran (0.3 ml)* | - | No deposition |
| 100 mg | Acetone (0.4 ml)* | - | No deposition |
| 100 mg | Toluene (2 ml)* | - | Oil out |
| 100 mg | Ethyl acetate (2 ml) | Hexane (5 ml) | Oil out |
| 100 mg | Dichloromethane (2 ml) | Hexane (5 ml) | Oil out |

### [Test Example 1] Measurement of powder X-ray diffraction

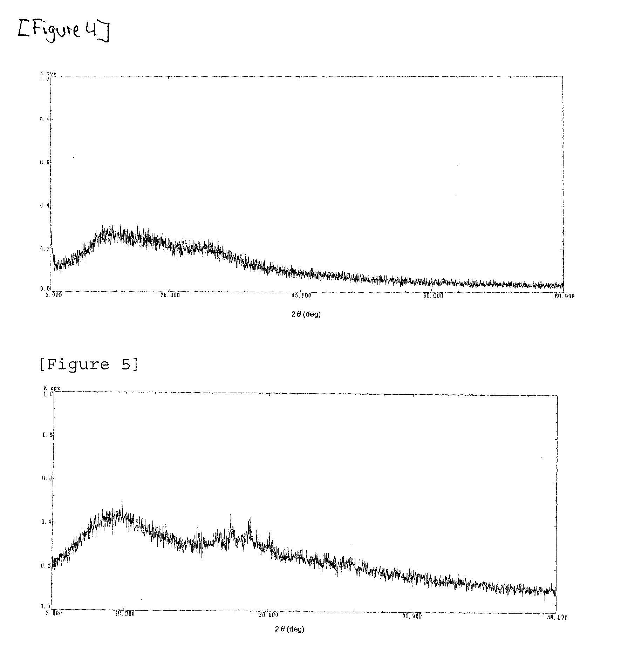

When the compound 1 and the compound 2 were prepared in a conventional method and powder X-ray diffraction was measured in the following conditions, crystalline diffraction peaks were not observed and the compound 1 and the compound 2 were confirmed to be amorphous. The X-ray diffraction pattern of the amorphous compound 2 was shown in Fig. 3, and that of the compound 1 was shown in Fig. 4. On the other hand, in the X-ray diffraction pattern of the compound 1 prepared in a method described in Example 1 of the present specification, a crystalline diffraction peak was observed as shown in Fig. 1 and the compound 1 was confirmed to be a crystal.

### [Test Example 2] Stability test in (sealed) condition at 60°C

Each test sample of an amorphous compound 1 prepared by a modification method, an amorphous compound 2 obtained by forming a free base of the amorphous compound 1 into hydrochloride in the same method as in Example 1 of Patent Literature 1, a crystal of the compound 1, which was prepared by the method described in Example 1 of the present specification, was charged into a brown glass bottle by each amount of 10 mg and preserved at 60°C, and the purities were compared after 4 weeks. As a result, the amorphous compound 1 was not observed to contain an impurity in an initial stage but the purity after 4 weeks was 99.4%. The purity of the amorphous compound 2 in the initial stage was 98.8% but decreased to 96.1% after 4 weeks, and an area value of an impurity peak at this time increased from 0.6% to 3.1%. On the other hand, the crystal of the compound 1 was not observed to contain an impurity in the initial stage and the purity was not changed also after 4 weeks. Not that the purity measurement was carried out in the following conditions.
- Column: Develosil CN-5 (Nomura Chemical), 250 mm x 4.6 mmϕ
- Mobile phase: 20 mM NaH₂PO₄ buffer (pH 6.9)/MeCN = 80/20
- Flow rate: 1.0 ml/min.
- Wavelength: UV 247 nm and 220 nm
- Charge amount: 10 µL
- Concentration of test sample: 100 µg/ml (H₂O)

It was shown from the above described result that the crystal of the compound 1 was significantly excellent from the viewpoint of stability as compared to the amorphous compound 1 and the amorphous compound 2.

**[Table 4]**

| | Purity (60°C, sealed) | |
|---|---|---|
| | Initial stage value | After 4 weeks |
| Amorphous compound 1 (free base) | 100.0% | 99.4% |
| Amorphous compound 2 (hydrochloride) | 98.8% | 96.1% |
| Crystal of compound 1 (free base) | 100.0% | 100.0% |

### [Test Example 3] Hygroscopicity test in condition of 66%RH (20°C)

Each test sample of an amorphous compound 1 prepared in a modification method, an amorphous compound 2 obtained by forming a free base of the amorphous compound 1 into hydrochloride in the same method as in Example 1 of Patent Literature 1, a crystal of the compound 1, which was prepared by the method described in Example 1 of the present specification, was charged into a desiccator at a humidity of 66%RH that was prepared by an aqueous saturated sodium nitrite solution by each amount of 100 mg and left stood at 20°C. Change in weights of each test sample was measured with time and a weight increase ratio was calculated. As a result, the amorphous compound 1 showed a weight increase ratio of 1.7% after 7 days and the purity at that time slightly decreased to be 99.8%. The amorphous compound 2 showed a weight increase ratio of 1.7% after 7 days and the purity at that time decreased from the initial value of 97.9% to 90.5%. On the other hand, the crystal of the compound 1 showed a weight increase ratio of 0.01% even after 7 days, hygroscopicity was not observed, and change in the purity was also not recognized. It was shown from the above described result that the crystal of the compound 1 was significantly excellent from the viewpoint of hygroscopicity as compared to the amorphous compound 1 and the amorphous compound 2.

**[Table 5]**

| | Weight increase ratio (66%RH, 20°C) | |
|---|---|---|
| | Initial stage value (purity) | After 7 days (purity) |
| Amorphous compound 1 (free base) | 0 (100.0%) | 1.7% (99.8%) |
| Amorphous compound 2 (hydrochloride) | 0 (97.9%) | 1.7% (90.5%) |
| Crystal of compound 1 (free base) | 0 (100.0%) | 0.01% (100.0%) |

It was found from all of these experimental results that, as compared to the amorphous compound 1 and the amorphous compound 2, the crystal of the compound 1 of the present invention has a high purity, is excellent in stability and has a characteristic of low hygroscopicity, and is therefore suitable for a use as medical drugs.

## Claims

1. A crystal of N-[2-({2-[(2S)-2-cyanopyrrolidine-1-yl]-2-oxoethyl}amino)-2-methyl propyl]-2-methylpyrazolo[1,5-a]pyrimidine-6-carboxamide (anagliptin).

2. The crystal according to claim 1, which has peaks around at 9.8°, 17.4°, 18.6°, 25.3°, and 25.8° as diffraction angles represented by 2θ in a powder X-ray diffraction pattern.

3. The crystal according to claim 1, wherein the diffraction angles represented by 2θ are substantially the same as the powder X-ray diffraction pattern shown in Fig. 1 described below.

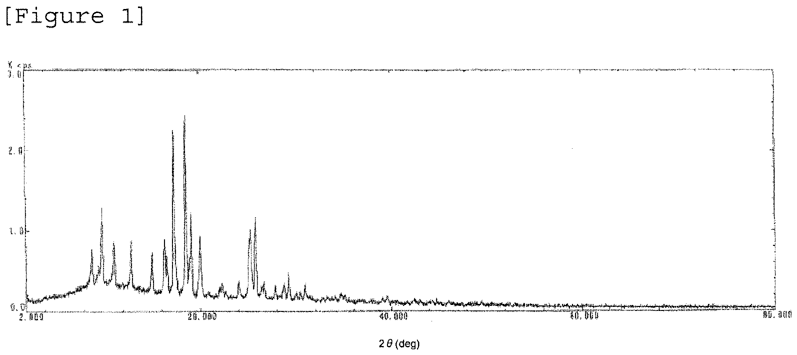

4. The crystal according to claim 1, which has absorption at wavenumbers of around 3340 cm⁻¹, 1664cm⁻¹, and 1654 cm⁻¹ in an infrared absorption spectrum.

5. The crystal according to claim 1, wherein the wavenumbers represented by cm⁻¹ are substantially the same as the infrared absorption spectrum shown in Fig. 2 described below.

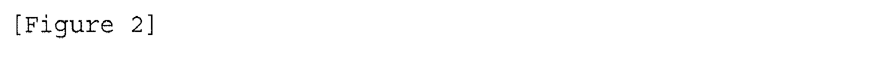

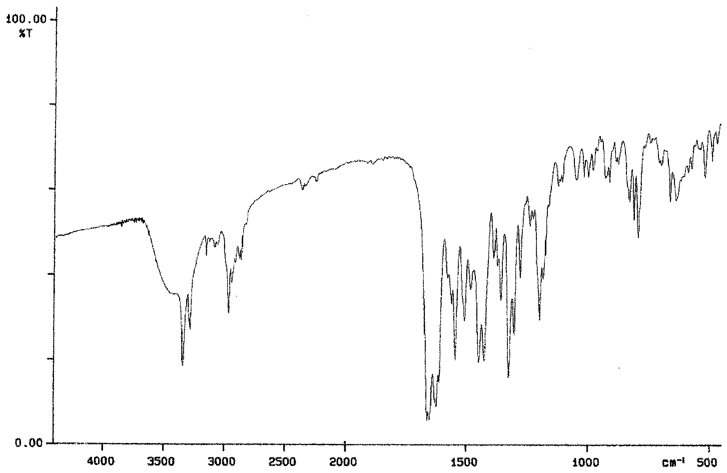

6. The crystal according to claim 1, which has a heat absorption peak around at 120°C in differential scanning calorimetry (DSC).

7. The crystal according to claim 1, wherein the heat absorption peak is substantially the same as the differential scanning calorimetry curve shown in Fig. 3 described below.

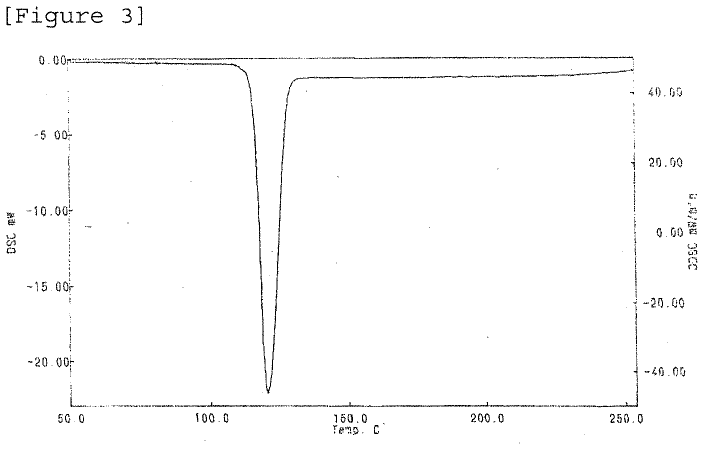

8. A method for producing a crystal of anagliptin, comprising reacting N-(2-amino-2-methylpropyl)-2-methylpyrazolo[1,5-a]pyrimidine-6-carboxamide and (2S)-N-chloroacetyl-2-cyanopyrrolidine in an organic solvent and thereafter crystallizing amorphia of anagliptin obtained by column chromatography purification from 2-propanol to obtain a crystal of anagliptin.

9. A method for producing a crystal of anagliptin, comprising reacting N-(2-amino-2-methylpropyl)-2-methylpyrazolo[1,5-a]pyrimidine-6-car boxamide and (2S)-N-chloroacetyl-2-cyanopyrrolidine in an organic solvent, dissolving the obtained amorphia of anagliptin in a crystallization solvent, and thereafter crystallizing by adding a seed crystal of anagliptin to obtain a crystal of anagliptin.

10. The method according to claim 8 or 9, wherein the reaction of N-(2-amino-2-methylpropyl)-2-methylpyrazolo[1,5-a]pyrimidine-6-car boxamide and (2S)-N-chloroacetyl-2-cyanopyrrolidine is carried out under the presence of alkaline carbonate and iodide.

11. The method according to claim 10, wherein the alkaline carbonate is potassium carbonate and the iodide is potassium iodide.

12. The method according to claim 8 or 9, wherein the organic solvent is selected from the group consisting of acetone, dichloromethane, and ethyl acetate.

13. The method according to claim 8 or 9, wherein (2S)-N-chloroacetyl-2-cyanopyrrolidine is 1 to 1.2 equivalent weight with respect to N-(2-amino-2-methylpropyl)-2-methylpyrazolo[1,5-a] pyrimidine-6-carboxamide.

14. The method according to claim 9, wherein the crystallization solvent is 2-propaol or ethyl acetate.

15. The method according to claim 9, wherein after the process of crystallization by adding the seed crystal of anagliptin to obtain a crystal of anagliptin, a process of crystallization by adding the seed crystal of anagliptin after concentration of the filtrate to obtain a crystal of anagliptin is further carried out plural times.
